# EUROPEAN PATENT SPECIFICATION

(11) **EP 2 417 948 B1**
(45) Date of publication and mention of the grant of the patent: **12.01.2022**
(21) Application number: 10755794.4
(22) Date of filing: 19.02.2010
(51) Int. Cl.: A61F 13/15, A61F 13/49, A61F 13/494

(54) **DISPOSABLE DIAPER**
WEGWERFWINDEL
COUCHE JETABLE

(30) Priority: 27.03.2009 JP 2009080272
(43) Date of publication of application: 15.02.2012
(73) Proprietor: Unicharm Corporation, Ehime 799-0111 (JP)
(72) Inventor: KUWANO, Seiichi, Kanonji-shi Kagawa 769-1602 (JP); ONO, Yoshio, Kanonji-shi Kagawa 769-1602 (JP)
(74) Representative: Staeger & Sperling Partnerschaftsgesellschaft mbB
(86) International application number: PCT/JP2010/052495
(87) International publication number: WO 2010/109993

(56) References cited:
- EP-A1- 2 260 812
- EP-A1- 2 482 777
- WO-A1-2007/015217
- JP-A- 2 065 859
- JP-A- 3 090 149
- JP-A- 3 198 851
- JP-A- 8 066 424
- JP-A- 2004 329 238
- US-A- 5 599 417
- US-A1- 2007 073 259

## Description

### {Technical Field}

The present invention relates to disposable diapers.

### {Background}

Conventionally, there are known disposable diapers having a pair of leakage-barrier cuffs extending in parallel in a longitudinal direction on respective sides of their crotch region.

For example, the pants-type diaper disclosed in JP 2002-102278 A1 (PTL 1) includes leg-openings annularly provided with leg elastic members on both sides of a crotch region and a pair of leakage-barrier cuffs extending in the longitudinal direction inside the respective leg-openings. The respective cuffs have elasticized edges so as to be elastically stretchable and contractible. These edges are elasticized by elastic members attached thereto under rectilinear tension. With this diaper put on the wearer's body, peripheral edges of the respective leg-openings are kept in close contact with the wearer's legs under the effect of the annular leg elastic members and the elasticized edges of the respective paired cuffs are kept in close contact with inguinal regions and/or inner sides of the wearer's thighs,

### {Citation List}

### {Patent Literature}

{PTL 1} JP 2002-102278 A1 Further prior art in this technical field is disclosed in documents EP 2 260 812 A1, EP 2 482 777 A1, WO 2007/015217 A1 and US 2007/073259 A1.

### {Technical Problem}

In the diaper disclosed in PTL 1, the leg elastic members are kept in close contact with the respective legs and at the same time the waist elastic members attached under tension to a peripheral edge of the waist-opening are kept in close contact with the waist to stabilize a position of the diaper on the wearer ' s. body in a vertical direction. In such a state of the diaper, the elasticized edges of the cuffs curved in a U-shape in the longitudinal direction are kept in close contact with the inguinal regions of the wearer from below and kept in close contact with inner sides of the wearer ' s thighs obliquely from below to prevent bodily fluids from leaking sideways in the crotch region. However, in diapers of relatively simple construction, there is a diaper which includes a pair of cuffs extending in parallel in the longitudinal direction immediately inside the respective leg-openings but not the elastic members annularly extending along the peripheral edges of the respective leg-openings. With the diaper of such construction put on the wearer's body, if the diaper slips down under a weight of body waste discharged thereon or due to a movement of the wearer's body, the cuffs' elasticized edges having been kept in close contact with the wearer's skin might also slip down and gaps might be formed between the skin and the cuffs' elasticized edges, and eventually bodily fluids might leak through these gaps,

An object of this invention is to improve the disposable diaper of prior art so that gaps apt to be formed between the leakage-barrier cuffs and the wearer's skin during use of the diaper may be effectively lessened.

### {Solution to Problem}

According to this invention, there is provided a disposable diaper having the features according to claim 1.

### {Advantageous Effects of Invention}

The disposable diaper according to this invention includes a pair of the leakage-barrier cuffs on each side of the crotch region. One of the paired cuffs is formed so as to extend outward beyond the bodily fluid absorbent core and includes the first elastic member attached to the outer side edge and the other of the paired cuffs has the proximal edge fixed to the inner surface thereof and includes the second elastic member attached the free edge extending in parallel to the proximal edge. Any of the paired cuffs includes the third elastic member attached under tension in the longitudinal direction thereto in the vicinity of the zone in which the proximal edge is fixed. With this diaper put on the wearer's body, one of the paired cuffs is kept in close contact with the wearer's skin, particularly with the inner side of the wearer's thigh, under the effect of the first elastic member and the other of the paired cuffs is kept in close contact with the inner side of the wearer's thigh under the effect of the second elastic member. In addition, these paired cuffs are kept in close contact with the wearer's skin also under the effect of the third elastic member. In consequence, even if the diaper slips down during use thereof, development of gaps between the paired cuffs and the wearer's skin should be reliably restricted.

### {Brief Description of Drawings}

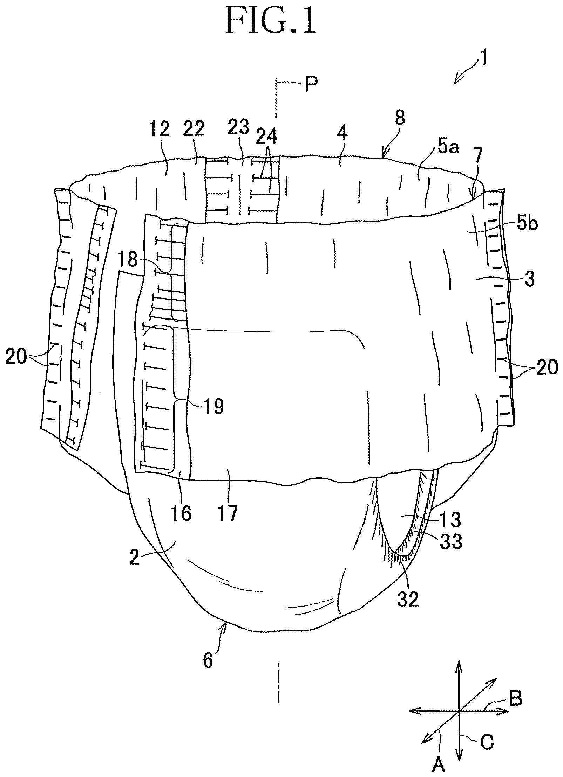

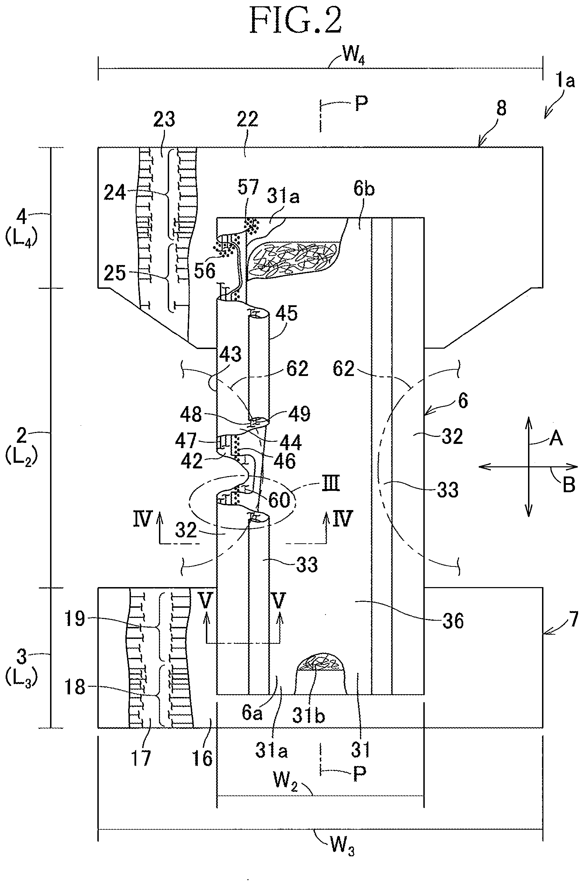

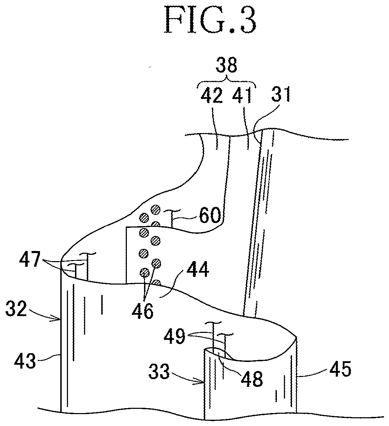

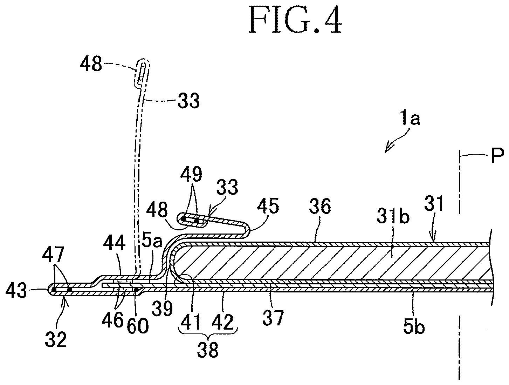

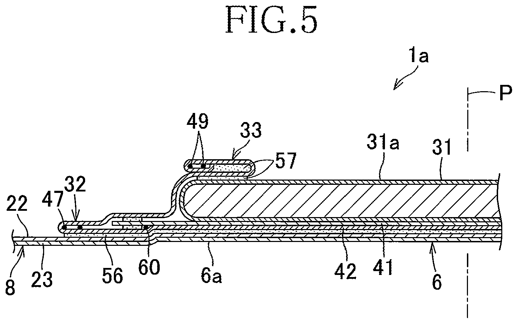

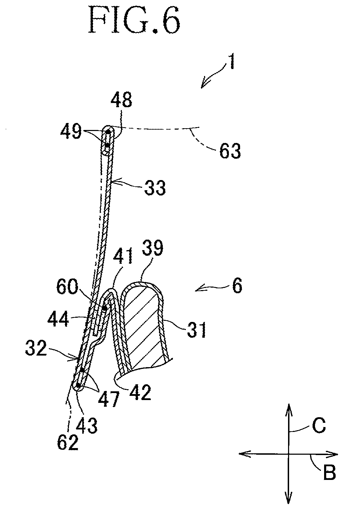

{Fig. 1} A partially cutaway perspective view of a disposable diaper.
{Fig. 2} A partially cutaway plan view of a developed diaper obtained from the diaper of Fig. 1.
{Fig. 3} A partially scale-enlarged diagram of Fig. 2.
{Fig. 4} A sectional view taken along line IV-IV in Fig. 2.
{Fig. 5} A sectional view taken along line V-V in Fig. 2.
{Fig. 6} A partial view of the diaper put on the wearer's body.

### {Description of Embodiments}

Details of the disposable diaper according to this invention will be described with reference to the accompanying drawings.

Fig. 1 is a partially cutaway perspective view of a pants-type disposable diaper 1 as one example of the disposable diaper wherein a longitudinal direction, a transverse direction and a height direction are indicated by double-headed arrows A, B, C being orthogonal to one another. The diaper 1 is symmetric about a center line P-P bisecting a dimension thereof in the transverse direction B and including a crotch panel 6 forming a crotch region 2, a front panel 7 forming a front waist region 3 extending forward from the crotch region 2 and a rear panel 8 forming a rear waist region 4 extending rearward from the crotch region 2 wherein the front and rear waist panels 7, 8 are put flat together along respective opposite laterals thereof and sealed at seams 20 extending continually in the height direction C. These front and rear panels 7, 8 cooperate with each other to define a waist-opening 12 and these front and rear panels 7, 8 cooperate together to define a pair of leg-openings 13. The front panel 7 is formed of a first inner sheet 16 and a first outer sheet 17 overlap with each other and bonded together with hot melt adhesives (not shown) intermittently applied thereto wherein these two sheets 16, 17 sandwich a plurality of elastic members 18 extending under tension across the front panel 7 in the transverse direction B and a plurality of elastic members 19 extending under tension on laterals of the front panel 7 but not fully extending across the front panel 7. These elasticmembers 18, 19 are attached to at least one of the sheets 16, 17 with hot melt adhesives (not shown) . The rear panel 8 is also formed of a second inner sheet 22 and a second outer sheet 23 overlap and bonded together with hot melt adhesives (not shown) wherein these two sheets 22, 23 sandwich a plurality of elastic members 24 extending under tension across the rear panel 8 and a plurality of elastic members 25 extending in the transverse direction B without fully extending across the rear panel 8 (See Fig. 2). These elastic members 24, 25 are attached to at least one of the sheets 22, 23 with hot melt adhesives (not shown) . In such diaper 1, a side facing the wearer' s skin (not shown) is designated herein as an inner side 5a and a side facing the wearer's garment (not shown) is designated herein as an outer side 5b.

Fig. 2 is a partially cutaway plan view of a developed diaper 1a obtained by peeling off the front panel 7 and the rear panel 8 of the diaper 1 joined together at the seams 20 in Fig. 1 from each other to develop these two panels 7, 8 in the longitudinal direction A and then stretching the crotch panel 6 and these front and rear panels 7, 8 in the longitudinal direction 1 as well as in the transverse direction B. In the developed diaper 1a, the crotch panel 6 has a rectangular shape which is relatively long in the longitudinal direction A wherein a section thereof overlapping the front panel 7 is bonded to the first inner sheet 16 with hot melt adhesives (not shown) and a section thereof overlapping the rear panel 8 is bonded to the second inner sheet 22 with hot melt adhesives (not shown). The crotch panel 6 is formed in its midsection in the transverse direction B with a bodily fluid absorbent structure 31 extending in the longitudinal direction A and formed on each side in the transverse direction B with an outer cuff 32 and an inner cuff 33 extending in parallel in the longitudinal direction A. The bodily fluid absorbent structure 31 includes a liquid-pervious cover sheet 31a and a bodily fluid absorbent core 31b wherein at least the inner surface side of entire surface of the core 31b is wrapped with the cover sheet 31a.

Fig. 3 is a scale-enlarged view showing a region encircled by imaginary line III in Fig. 2. The outer cuff 32 and the inner cuff 33 are formed of an outer sheet 42 (See Fig. 4 also) and the outer sheet 42 is folded back to define two layers which are locally joined together along a joint zone 44 by means of one of hot melt adhesives 46 and sealing processing wherein the joint zone 44 extends in parallel to the center line P-P fully between a front end 6a and a rear end 6b of the crotch panel 6 (See Fig. 2). The outer cuff 32 is defined outside the joint zone 44 as viewed in the transverse direction B of the crotch panel 6 and the inner cuff 33 is defined inside the joint zone 44 wherein the joint zone 44 defines a boundary zone between the outer cuff 32 and the inner cuff 33. The outer sheet 42 has boundary zone's elastic member 60 attached thereto under tension so as to extend in parallel to the center line P-P in the longitudinal direction A in the vicinity of the joint zone 44. The expression "in the vicinity of the joint zone 44" used herein means that the boundary zone's elastic member 60 is spaced from the joint zone 44 in the transverse direction B within 10mm, more preferably within 5mm. In an example of the infant diapers using the boundary zone's elastic member 60 in this manner has, in the longitudinal direction A indicated in Fig. 2, a dimension L₂ of the crotch region 2 in a range of 200 to 300mm, a dimension L₃ of the front waist region 3 in a range of 100 to 150mm and a dimension L₄ of the rear waist region 4 in a range of 100 to 150mm and, in the transverse direction B, a dimension W₂ in a range of 150 to 200mm, a dimension W₃ of the front waist region 3 in a range of 300 to 400mm and a dimension W₄ of the rear waist region 4 in a range of 300 to 400mm. A liquid-impervious inner sheet 41 is sandwiched between the outer sheet 42 and the bodily fluid absorbent structure 31 as viewed in the thickness direction of the bodily fluid absorbent structure 31.

Fig. 4 is a sectional view taken along line IV-IV in Fig. 2. In the crotch panel 6, the bodily fluid absorbent structure 31 has an inner surface 36 adapted to come in contact with skin of the wearer (not shown) and an opposite surface 37 which faces, in turn, a leakage-barrier sheet 38 (See also Fig. 3). The leakage-barrier sheet 38 composed of the inner sheet 41 formed of a liquid-impervious plastic film and the outer sheet 42 formed of a nonwoven fabric, more preferably, a nonwoven fabric formed of water-repellent fibers, most preferably, an air-permeable and liquid-impervious nonwoven fabric. The inner sheet 41 is bonded to the opposite surface 37 of the bodily fluid absorbent structure 31 with hot melt adhesives (not shown) and extends across the bodily fluid absorbent structure 31 in the transverse direction B beyond side edges 39 thereof. The outer sheet 42 is bonded to the inner sheet 41 with hot melt adhesives (not shown) to define the outer side 5b of the diaper 1 in the crotch region 2 and extends across the bodily fluid absorbent structure 31 in the transverse direction B beyond the side edges 39 of the bodily fluid absorbent structure 31. The outer sheet 42 extends in the transverse direction B beyond the side edges of the inner sheet 41 and is folded back toward the center line P-P along a first folded zone 43 so as to overlap itself. The outer sheet 42 overlapping itself in this manner is bonded to itself with hot melt adhesives 46 in the joint zone 44. The outer sheet 42 having been folded back is folded back again along a second folded zone 45 closer to the center line P-P than the first folded zone 43 so as to extend outward in the transverse direction B and a distal end thereof forms a sleeve 48 covering inner side elastic members 49. The inner side elastic members 49 extend in the longitudinal direction A in parallel to the center line P-P and bonded under tension to the inner surface of the sleeve 48 with hot melt adhesives (not shown). The outer sheet 42 is somewhat sleeve-like also between the first folded zone 43 and the joint zone 44 and outer side elastic members 47 are attached under tension in the longitudinal direction A in parallel to the center line P-P to the inner surface of the first folded zone 43 with hot melt adhesives (not shown) . In such crotch panel 6, a segment of the outer sheet 42 extending between the first folded zone 43 and the joint zone 44 defines the outer cuff 32 adapted to be kept in close contact with the inner sides of the wearer's thighs and the first folded zone 43 defines the outer side edge of this outer cuff 32. A segment extending between the joint zone 44 and the sleeve 48 defines the inner cuff 33 adapted to be kept in close contact with the inner sides of the wearer's thighs and the joint zone 44 defines a proximal edge of the inner cuff 33 and the sleeve 48 defines a free edge adapted to be deformed at a relatively high degree of freedom (See Figs. 2 and 3) . Such inner cuff 33 is formed by an extension of the outer cuff 32 lying on the inner side 5a of the diaper 1 so that the inner cuff 33 may rise from the inner side 5a as indicated by imaginary lines when the diaper 1 of Fig. 1 is assembled from the developed diaper 1a.

Fig. 5 is a sectional view taken along line V-V in Fig. 2, showing a construction of the front end 6a of the crotch panel 6. In the front end 6a, the outer sheet 42 underlying the bodily fluid absorbent structure 31 as seen in Fig. 5 is bonded to the second inner sheet 22 with hot melt adhesives 56 and the outer cuff 32 is also bonded to the second inner sheet 22. In the inner cuff 33 in a folded state, the outer sheet 42 is bonded to itself and bonded also to the cover sheet 31a with hot melt adhesives 57. In the rear end 6b (See Fig. 2) of the crotch panel 6 also, the outer sheet 42 and the outer cuff 32 formed of this outer sheet 42 are bonded to the first inner sheet 16 in the same manner as those in the front end 6a. The inner cuff 33 is folded back in the similar manner to that in Fig. 4. One of the outer side elastic members 47, the inner side elastic member 49 and the boundary zone's elastic member 60, more preferably, the boundary zone's elastic member 60 extends in a stretchable and contractible manner across the elastic members 19, 25 in the front and rear panels 7, 8. These elastic members extending across one another cooperate together to surround the associated leg of the wearer and thereby to function in the same manner as the leg elastic members annularly arranged.

Fig. 6 is a schematic sectional view of the crotch panel 6 after the developed diaper 1a constructed as shown in Figs. 2 through 5 has been assembled to the diaper 1 of Fig. 1, wherein some of the members are not shown for convenience of illustration but the positions of these members are the same as the positions in the sectional view of Fig. 4. In Fig. 6, a portion of the inner side 62 of the wearer's thigh and a portion of the inguinal region being contiguous thereto are indicated by imaginary lines, respectively. In the longitudinal direction A, the crotch panel 6 of the diaper 1 bows in a U-shape and is suspended at the front end 6a and the rear end 6b from the front panel 7 and the rear panel 8, respectively. In the transverse direction B, the crotch panel 6 bows to be convex downward of the diaper 1. Upon contraction of the outer side elastic members 47 and the inner side elastic members 49, the outer cuff 32 and the inner cuff 33 extend upward from the respective side edges 39 of the bodily fluid absorbent structure 31. In consequence, the first folded zone 43 of the outer sheet 42 defining the lower edge of the outer cuff 32 as viewed in Fig. 6 comes in contact with the inner side 62 of the wearer's thigh, and the second folded zone 45 of the inner cuff 33 is unfolded so that the sleeve 48 now defining the upper edge of the inner cuff 33 may come in contact with the inner side 62 of the wearer's thigh or the inguinal region 63. In this way, these paired outer cuffs 32 and inner cuffs 33 respectively function as leakage-barrier cuffs adapted to prevent bodily fluids from leaking sideways from the diaper 1. Along the joint zone 44 in the outer sheet 42 defining the proximal edge of the inner cuff 33, the outer cuff 32 and the inner cuff 33 are branched from each other and the joint zone 44 is elastically kept in contact with the inner side 62 of the wearer's thigh under the effect of the boundary zone's elastic member 60.

With the diaper 1 put on the wearer's body in a preferred manner, the inner side 62 of the wearer' s thigh is at the position indicated by the imaginary lines 62 in Fig. 2 and the outer side elastic members 47, the inner side elastic members 49 and the boundary zone's elastic member 60 lying at the bottom of the crotch region 6 as viewed in Fig. 1 are stretched and deformed inward in the transverse direction B so as to get close to the center line P. The outer side elastic members 47 of the outer cuff 32 and the inner side elastic members 49 of the inner cuff 33 are elastically put in contact with the inner side 62 of the wearer's thigh in the longitudinal direction C of the diaper 1 as well as in the transverse direction B extending from the inner side toward the outer side of the diaper 1 but these elastic members do not annularly tighten the wearer's thigh. Assuming that the outer cuff 32 and the inner cuff 33 have no additional features, the first folded zone 43 in the outer cuff 32 and the sleeve 48 of the inner cuff 33 would be spaced from the inner side 62 of the wearer's thigh when the diaper 1 unintentionally slips down along the wearer's body so as to develop gaps between these cuffs 32, 33 and the inner side 62 of the wearer's thigh, causing bodily fluids to leak sideways through such gaps and deteriorating the desired function of the outer cuff 32 and the inner cuff 33 as the leakage-barrier cuffs. However, the outer sheet 42 in the diaper 1 includes the boundary zone's elastic member 60 provided in the vicinity of the joint zone 44 about which the outer cuff 32 and the inner cuff 33 are branched from each other functions to keep the outer cuff 32 and the inner cuff 33 in close contact with the inner side 62 of the wearer's thigh in the joint zone 44. In this way, a movement of the outer cuff 32 away from the inner side 62 of the wearer's thigh due to unintentional slip down of the diaper 1 can be restricted by cooperation of the outer side elastic members 43 and the boundary zone's elastic member 60, and a movement of the inner cuff 33 away from the inner side 62 of the wearer's thigh can be restricted by cooperation of the inner side elasticmembers 49 and the boundary zone's elastic member 60 to prevent the gaps causing leakage of bodily fluids from developing between these cuffs 32, 33 and the inner side 62 of the wearer's thigh. While the boundary zone's elastic member 60 may be bonded to the outer cuff 32 or the inner cuff 33 in the vicinity of the joint zone 44, this elastic member 60 is bonded to the outer sheet 42 at a position inside the joint zone 44, more specifically, at a position defined between the joint zone 44 and the center line P-P as exemplarily shown in Figs. 2 and 4. While the boundary zone's elastic member 60 is not limited to the illustrated example and according to the invention it is formed of a plurality of rubber threads, it is preferred in any cases that a force F₀ which is needed to stretch the joint zone 44 from a state of under contraction of the boundary zone's elastic member 60 by 20% should be higher than a force F₂ which is needed to stretch the inner side elastic members 49 from the state thereof under contraction by 20% rather than a force F₁ which is needed to stretch the outer side elastic members 47 from the state thereof under contraction by 20% and the inner cuff 33 can be prevented from moving away from the wearer's skin. If any elastic members alternative to the boundary zone's elastic member 60 are attached to the outer sheet 42 at a position spaced inward from the joint zone 44, for example, in the infant diaper 1, at a position spaced inward from the joint zone 44 by 10mm or more, an amount by which such elastic members can be stretched toward the center line P-P will be restricted by the inner side 62 of the wearer' s thigh in comparison to the exemplarily illustrated boundary zone's elastic member 60. Such elastic members being alternative to the boundary zone's elastic member 60 are undesirable because the closer to the midsection of the diaper 1 the elastic members being alternative to the boundary zone's elastic member 60 are located, the lower the force keeping the cuffs in close contact with the inner side 62 of the wearer's thigh in the transverse direction B becomes and the effect to restrict the movement of the outer cuff 32 and the inner cuff 33 will be correspondingly deteriorated. While this invention has been described above exemplarily based on the pants-type disposable diaper 1, this invention is applicable also to a so-called open-type diaper having the front waist region 2 and the rear waist region 3 are detachably connected to each other by means of fastener.

### {Reference Signs List}

- 1: disposable diaper
- 2: crotch region
- 3: front waist region
- 4: rear waist region
- 5a: inner surface
- 5b: outer surface
- 18: elastic members
- 19: elastic members
- 24: elastic members
- 25: elastic members
- 31a: liquid-pervious sheet (cover sheet)
- 31b: core
- 32: cuffs (outer cuffs)
- 33: cuffs (inner cuffs)
- 43: outer side edges (first folded zones)
- 44: proximal edges (joint zones)
- 47: first elastic members (outer side elastic members)
- 49: second elastic members (inner side elastic members)
- 60: third elastic members (boundary zones' elastic members)
- A: longitudinal direction
- B: transverse direction
- P: center line

## Claims

1. A disposable diaper (1) having a longitudinal direction, a transverse direction and a thickness direction being orthogonal to one another and comprising a front waist region (3) extending forward in the longitudinal direction, a rear waist region (4) extending rearward in the longitudinal direction, and a crotch region (2) extending in the longitudinal direction between the front waist region and the rear waist region, wherein the crotch region (2) is formed with a bodily fluid absorbent core (31b) having a surface at least partially covered with a liquid-pervious sheet (13a) and the crotch region (2) comprises a leakage-barrier sheet (38) composed of an inner sheet (41) and an outer sheet (42) and is formed along respective side edges thereof opposed to each other in the transverse direction with a pair of stretchable and contractible leakage-barrier cuffs (32, 33) which are formed of the outer sheet (42) which is folded over itself and bonded to the inner sheet (41) along a joint zone (44) and extend parallel outside the core as viewed in the longitudinal direction, **characterized in that**:
the front waist region (3), the rear waist region (4) and the crotch region (2) respectively have an inner side facing the wearer's skin and an outer side opposed to the inner side; wherein
• one of the paired cuffs, being the outer cuff (32) is formed so as to extend outward beyond the core (31b) in the transverse direction and includes
- the inner side, the outer side,
- an outer side edge (43) extending in parallel to a center line extending in the longitudinal direction so as to bisect a dimension of the crotch region in the transverse direction and at least
- one outer side elastic members (47) attached under tension to the outer side edge (43) so as to make the outer side edge (43) stretchable and contractible in the longitudinal direction;
• the other of the paired cuffs, being the inner cuff (33), includes
- the joint zone (44) defining a proximal edge fixed to the inner side of the one of the paired cuffs (32) so as to extend in parallel to the center line in the longitudinal direction,
- a free edge (48) extending in parallel to the proximal edge and at least one inner side elastic members (49) attached under tension to the free edge (48) so as to make the free edge (48) stretchable and contractible in the longitudinal direction;
- at least one of the paired cuffs (32) includes a boundary zone's elastic member (60) extending in the longitudinal direction and attached under tension at a position inside the joint zone (44) defining the proximal edge ; and
- the paired cuffs (32, 33) respective have opposite ends as viewed in the longitudinal direction fixed to the inner side of the diaper (1),
- wherein the boundary zone's elastic member (60) is bonded to the outer sheet (42) at a position inside the joint zone (44),
- wherein along the joint zone (44) in the outer sheet (42) defining the proximal edge of the inner cuff (33), the outer cuff (32) and the inner cuff (33) are branched from each other and the joint zone (44),
- wherein the boundary zone's elastic member (60) is formed of a plurality of rubber threads; and with the diaper put on the wearer's body, the joint zone (44) is configured to be elastically kept in contact with the inner side (62) of the wearer's thigh under the effect of the boundary zone's elastic member (60).

## Patentansprüche

1. Wegwerfwindel (1), die eine Längsrichtung, eine Querrichtung und eine Dickenrichtung, die orthogonal zueinander sind, aufweist und einen vorderen Taillenbereich (3), der sich in der Längsrichtung nach vorne erstreckt, einen hinteren Taillenbereich (4), der sich in der Längsrichtung nach hinten erstreckt, und einen Schrittbereich (2), der sich in der Längsrichtung zwischen dem vorderen Taillenbereich und dem hinteren Taillenbereich erstreckt, umfasst, wobei der Schrittbereich (2) mit einem eine Körperflüssigkeit absorbierenden Kern (31b) ausgebildet ist, der eine Oberfläche aufweist, die mindestens teilweise mit einer flüssigkeitsdurchlässigen Lage (13a) bedeckt ist, und der Schrittbereich (2) eine Auslaufsperrlage (38) umfasst, die aus einer inneren Lage (41) und einer äußeren Lage (42) zusammengesetzt ist und entlang ihrer in der Querrichtung einander gegenüberliegenden jeweiligen Seitenborten mit einem Paar dehnbarer und zusammenziehbarer Auslaufsperrbündchen (32, 33) ausgebildet ist, die aus der äußeren Lage (42) gebildet sind, die auf sich selbst umgefaltet und mit der inneren Lage (41) entlang einer Verbindungszone (44) verbunden ist, und die sich in Längsrichtung gesehen parallel außerhalb des Kerns erstrecken, **dadurch gekennzeichnet, dass**:
der vordere Taillenbereich (3), der hintere Taillenbereich (4) und der Schrittbereich (2) jeweils eine der Haut des Trägers zugewandte Innenseite und eine der Innenseite gegenüberliegende Außenseite aufweisen; wobei
• eines der paarweisen Bündchen, nämlich das äußere Bündchen (32), so ausgebildet ist, dass es sich über den Kern (31b) hinaus in der Querrichtung nach außen erstreckt und umfasst:
- die innere Seite, die äußere Seite,
- eine äußere Seitenborte (43), die sich parallel zu einer Mittellinie erstreckt, die sich in der Längsrichtung so erstreckt, dass sie eine Abmessung des Schrittbereichs in der Querrichtung halbiert, und mindestens
- ein elastisches Außenseitenelement (47), das unter Spannung an der äußeren Seitenborte (43) befestigt ist, um die äußeren Seitenborte (43) in der Längsrichtung dehnbar und zusammenziehbar zu machen;
• das andere der paarweisen Bündchen, nämlich das innere Bündchen (33), umfasst:
- die Verbindungszone (44), die eine proximale Borte definiert, die an der Innenseite des einen der paarweisen Bündchen (32) so fixiert ist, dass sie sich parallel zur Mittellinie in der Längsrichtung erstreckt,
- eine freie Borte (48), die sich parallel zur proximalen Borte erstreckt, und mindestens ein innenseitiges elastisches Element (49), das unter Spannung an der freien Borte (48) befestigt ist, um die freie Borte (48) in Langsrichtung dehnbar und zusammenziehbar zu machen;
- dass mindestens eines der paarweisen Bündchen (32) ein elastisches Grenzzonenelement (60) umfasst, das sich in der Längsrichtung erstreckt und unter Spannung an einer Position innerhalb der die proximale Borte definierenden Verbindungszone (44) befestigt ist; und
- die paarweisen Bündchen (32, 33) jeweils in Längsrichtung gesehen gegenüberliegende Enden haben, die an der Innenseite der Windel (1) fixiert sind,
- wobei das elastische Grenzzonenelement (60) an einer Position innerhalb der Verbindungszone (44) mit der äußeren Lage (42) verbunden ist,
- wobei entlang der Verbindungszone (44), in der die proximale Borte des inneren Bündchens (33) definierenden äußeren Lage (42), das äußere Bündchen(32) und das innere Bündchen (33) voneinander und von der Verbindungszone (44) abgezweigt sind,
- wobei das elastische Grenzzonenelement (60) der aus einer Mehrzahl von Gummifäden gebildet ist; und wenn die Windel am Körper des Trägers angelegt ist, die Verbindungszone (44) so konfiguriert ist, dass sie unter der Wirkung des elastischen Grenzzonenelements (60) mit der Innenseite (62) des Oberschenkels des Trägers elastisch in Kontakt gehalten wird.

## Revendications

1. Couche jetable (1) ayant une direction longitudinale, une direction transversale et une direction d'épaisseur qui sont orthogonales entre elles et comprenant une région de taille avant (3) s'étendant vers l'avant dans la direction longitudinale, une région de taille arrière (4) s'étendant vers l'arrière dans la direction longitudinale, et une région d'entrejambe (2) s'étendant dans la direction longitudinale entre la région de taille avant et la région de taille arrière, dans laquelle la région d'entrejambe (2) comporte un noyau d'absorption de fluides corporels (31b) ayant une surface au moins partiellement recouverte d'une feuille perméable aux liquides (13a) et la région d'entrejambe (2) comprend une feuille de barrière contre les fuites (38) composée d'une feuille intérieure (41) et d'une feuille extérieure (42) et comporte le long des bords latéraux respectifs de celle-ci opposés l'un à l'autre dans la direction transversale une paire de parements de barrière contre les fuites, étirables et rétractables (32, 33) qui sont constitués de la feuille extérieure (42) qui est repliée sur elle-même et collée à la feuille intérieure (41) le long d'une zone de liaison (44) et s'étendent parallèlement à l'extérieur du noyau tel que vu dans la direction longitudinale, **caractérisée par le fait que** :
la région de taille avant (3), la région de taille arrière (4) et la région d'entrejambe (2) ont respectivement un côté intérieur faisant face à la peau du porteur et un côté extérieur opposé au côté intérieur ; où
• l'un des parements appariés, qui est le parement extérieur (32), est formé de façon à s'étendre vers l'extérieur au-delà du noyau (31b) dans la direction transversale et comprend
- le côté intérieur, le côté extérieur,
- un bord côté extérieur (43) s'étendant parallèlement à une ligne centrale s'étendant dans la direction longitudinale de façon à diviser en deux une dimension de la région d'entrejambe dans la direction transversale, et
- au moins un élément élastique côté extérieur (47) attaché sous tension au bord côté extérieur (43) de façon à rendre le bord côté extérieur (43) étirable et rétractable dans la direction longitudinale ;
• l'autre des parements appariés, qui est le parement intérieur (33), comprend
- la zone de liaison (44) définissant un bord proximal fixé au côté intérieur du premier (32) des parements appariés de façon à s'étendre parallèlement à la ligne centrale dans la direction longitudinale ;
- un bord libre (48) s'étendant parallèlement au bord proximal et à au moins un élément élastique côté intérieur (49) attaché sous tension au bord libre (48) de façon à rendre le bord libre (48) étirable et rétractable dans la direction longitudinale ;
- au moins l'un (32) des parements appariés comprend un élément élastique de zone limite (60) s'étendant dans la direction longitudinale et attaché sous tension à une position à l'intérieur de la zone de liaison (44) définissant le bord proximal ; et
- les parements appariés (32, 33) ont des extrémités opposées respectives tel que vu dans la direction longitudinale fixées au côté intérieur de la couche (1) ;
- dans laquelle l'élément élastique de zone limite (60) est collé à la feuille extérieure (42) à une position à l'intérieur de la zone de liaison (44) ; dans laquelle, le long de la zone de liaison (44) dans la feuille extérieure (42) définissant le bord proximal du parement intérieur (33), le parement extérieur (32) et le parement intérieur (33) se ramifient l'un à partir de l'autre et à partir de la zone de liaison (44) ;
- dans laquelle l'élément élastique de zone limite (60) est constitué d'une pluralité de fils de caoutchouc et, avec la couche mise sur le corps du porteur, la zone de liaison (44) est configurée pour être maintenue élastiquement en contact avec le côté intérieur (62) de la cuisse du porteur sous l'effet de l'élément élastique de zone limite (60).
